# EUROPEAN PATENT SPECIFICATION

(11) **EP 0 787 427 B1**
(45) Date of publication and mention of the grant of the patent: **05.09.2001**
(21) Application number: 97300644.8
(22) Date of filing: 31.01.1997
(51) Int. Cl.: A01K 29/00, A01K 67/02, A61F 6/14

(54) **Intrauterine device for improving fattening in mammals**
Intrauterine Vorrichtung zur Steigerung der Gewichtszunahme bei Säugetieren
Dispositif intrautérine pour l'amélioration de l'engraissage des mammifères

(30) Priority: 01.02.1996 AR 33528496
(43) Date of publication of application: 06.08.1997
(73) Proprietor: Turin, Enrique Horacio, (2700) Pergamino, Province of Buenos Aires (AR)
(72) Inventor: Turin, Enrique Horacio, (2700) Pergamino, Province of Buenos Aires (AR)
(74) Representative: Jenkins, Peter David

(56) References cited:
- EP-A- 0 117 163
- EP-A- 0 614 605
- DE-A- 2 925 993
- DE-C- 4 237 950
- FR-A- 2 290 906
- FR-A- 2 445 139
- GB-A- 2 079 158
- GB-A- 2 154 875

## Description

The present invention relates to an intrauterine device for improving fattening in mammals with two separated uterine horns. The intrauterine device, when used, functions by altering the normal information existing between both uterine horns and the ovaries of domestic female mammals, with the object of making a hormonal alteration leading to the suppression of the estrous cycle, the absence of ovulation, and the increase in the daily weight gain particularly of cows and heifers.

Intrauterine devices (IUD) are the most efficient birth-control method in humans (World Health Organization, 1987). Their mechanism, which consists in suppressing human fertility, has been subject to controversy because of the diversity of methods found in trials undergone by animals of different species (Marston and Kelly, 1966).

As far as women and non-human primates are concerned, the IUD interferes with fertilization while the menstrual cycle remains undisturbed (Chi, 1993).

Rodent ovulation, spermatic transport and fertilization are unaltered by IUD action, however it interferes with the process of implantation by means of these three mechanisms:
a) embryonic degeneration,
b) acceleration of embryonic transit and ejection,
c) alteration of the implanting uterine component or decidua (Marston and Kelly, 1996).

In rodents with two uterine horns and two independent uterine necks, such as rats and rabbits, anti-implantation effects are reached when a IUD is placed in each horn (Davis, 1972). In the case of mice, both horns are joined by a common neck, that is why the insertion of a IUD in one horn is sufficient so as to impede the embryonic implantation in both horns (Marston and Kelly, 1966).

In relation with rodents and ruminants having two independent uterine horns, contraceptive effects are reached only when a IUD is placed in each horn (Marston and Kelly, 1966. Davis 1972, Hawk et al 1973).

In the case of ruminants such as sheep and cows apart from contraceptive effects, there is an alteration of the oestrous cycle according to the distention degree caused by the IUD in the uterus (Nalbandov et al., 1955: Hansel & Wagner, 1960; Turin et al., 1966).

The insertion of a IUD in sheep interferes with the oestrous periodicity without restraining ovulation. The contraceptive effect, in this case, seems to be the result of an alteration in the spermatic transport and an early luteinization. The origin of this phenomenon is possibly related to the uterine distention after the insertion of a IUD, these circumstances are manifested when the IUD is placed homolaterally with respect to the ovulating ovary.

Specialized literature suggests that the insertion of IUD in cows does not modify the oestrous intensity, but it has a contraceptive effect by means of an alteration in the spermatic transport and an early luteinization caused by uterine distention. When talking about contraceptive efficiency, size and location of IUDs seem to be key factors (Marston and Kelly, 1966; Hawk et al.), 1968)

The method consists in inserting (by means of a specially designed applicator) at least one device into the uterine cavity, particularly one in each uterine horn, at least.

According to what is experimentally known, there is a variation according to the species regarding the IUD contraceptive effect on most mammal species having mechanisms that control the effects against fertility.

Human and non-human primates have a menstrual cycle which is unaltered by lUDs and their natural ovulation cycles remain unaffected. The uterine device applied according to the present invention interferes with the oestrous period since it affects the ovarian function. The effects of this device seem to be critical for ruminants because it increases the uterine distention occurring in the uterus after it is located in one or both uterine corns. The following description relates to the effects of a copper bearing device specially designed for altering the ovarian function, reproductive capacity and weight gain of cows and heifers.

Intents of weight gain were effected in the past based on animal castration. In 1906, Dutto affirmed that Nuffer was the first one to employ surgical practices with ulterior ovary ectomy. Furthermore Dutto also reported bloody techniques carried out in the past by Charlier and Degive. Dutto also designed the ovarytome, an instrument which improved Degive's cruel technique of bonding ovaries This technique is still being used at present in spite of the fact that not only acts in a bloody manner, but also has a variable success depending on the ovary bond position and its peritoneal revascularization.

It is known that weight gain is one of the objects of our cattle breeding However This method consisting of intrauterine devices can only be applicable to the females. Female bovine animals have what is called sexual cycle, which is central to coition. This cycle involves four phases, the oestrum period, also called mating season, which lasts 24 hours approximately and is the only time the female is willing to mate, the precedent period is called pro-oestrum and, in case coition does not result in conception, there is a following phase which is called metoestrum, afterwards a pause called diostrum period takes place, and then a new cycle starts. Specific hormones are involved so as to reach a normal and timely completion of the cycle

Ovulation takes place during the oestrum period in which the mature ovule is released by the ovary. Once the ovulation has started, the *luteuos corpus* is formed in the ovary. This corpus is in charge of producing Progesterone, a hormone of important anabolic effects in charge of controlling coming cycles and pregnancy normal development. In case conception is unfruitful, the *luteous corpus* is disintegrated by a hormone called Prostaglandine F2, and thus a new sexual cycle starts.

The synthesis of Prostaglandine F2 alpha occurs in the uterus, more precisely in the endometrium, then it reaches the *luteous corpus* by means of a mechanism called uterus-ovary crosscurrent which disintegrates it before the beginning of a new cycle.

In view of the above, it should be mentioned that unless Prostaglandine F2 alpha is present, the *luteous corpus* will not be disintegrated and will continuously generate Progesterone for an undetermined period causing important effects:
- absence of oestrum, ovulation and conception caused by a suprabasal level of Progesterone generated by persistent *luteous* structures. This suprabasal level blocks the beginning of a new cycle by inhibiting the follicle hormones (unovulation)
- anabolic effect: the techa luteinization alters the techa/granular relation and the excessive substance produced by the luteinizated internal theca (hypertrophy) fails to aromatize in granulose and to turn into estrogen. Consequently, androgens are released increasing the concentration six or seven times, varying from 0.026 to 0.163 nanograms/ml. Thus, these androgens are responsible of weight gain.

The present invention provides an intrauterine device for improving fattening in mammals with two separated uterine horns, the device comprising a main elongated body which is coiled by a metallic filament and has a lower extreme being formed by two or more arms which are inverted "V" shaped, characterised in that the main elgonated body has an upper extreme being formed by a head which has an upper pyramidal, rounded or oval surface and each arm is provided with a longitudinal slit.

DE-A-2925993 discloses an IUD for human use.

EP-A-0614605 discloses an IUD to improve fattening in mammals.

In use, two devices are implanted so as to extend into each of the uterine horns of the bovine uterine cavity, in order to block the normal function existing between the uterus and the ovaries, thus preventing Prostaglandina F2 alpha from synthesizing.

Embodiments of the present invention will now be described by way of example only, with reference to the accompanying drawings, in which:-
Figure 1 is an elevational side view of one embodiment of the device.
Figure 2 shows a second embodiment of the device, and
Figure 3 shows a third embodiment of the device.

References 1, 2 and 3 corresponding to the above mentioned figures stand for the head (1), the body (2) and the arms (3) of the device respectively.

It is important to bear in mind that the slit at the lower surface of each arm serves the purpose of avoiding material fatigue and causing a snapping effect to keep the arms open. The function performed by the arms is to provide force and contact with the endometrium of the uterus of the cow so as to obtain the desired results.

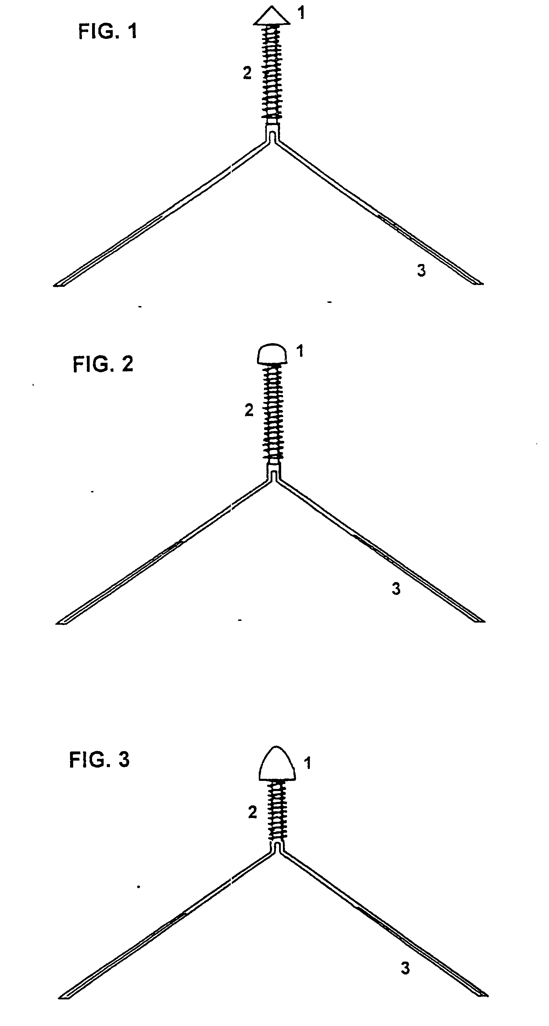

It should be pointed out that the device in Figure 1 is intended for cows and heifers and the devices in Figures 2 and 3, which are bigger, are intended for dairy cows having larger uterus. A pyramidal, rounded or oval head is located in the other extreme of the main body. The uterine device corresponding to the present invention is intended for influencing cow fattening rhythm and handling.

Basically, the functional characteristic of this device is to remain inside the uterus of the mammal in order to cause a persistent irritation in the endometrium which is complemented by the action of the copper (metallosys) and the distention of the walls of uterine horns. This way. the synthesis of Prostaglandine F2 would be avoided, causing the animal to have a period of oestral absence, being the *luteus corpus* persistent, Progesterone is consequently released by this structure.

This involves three basic steps:
1. Alteration of the normal functioning of the endometrium cell due to copper action which releases ions,
2. Irritation by mechanic action, and
3. Distention of the walls of the uterine horns.

In order to facilitate the understanding of the constitutive, functional and emplacement characteristics of the method described in the present invention, an embodiment example is described below and shown schematically in the attached sheet without specific scale, with the precise explanation regarding the non-restrictive or exclusive character of the example according to the scope of protection of the present invention, in contrast, the example is to be considered only as merely explanatory of the fundamental conception on which the invention is based.

The constitutive components of the present invention are (1) a head, (2) a body, and (3) two arms, as shown in Figures 1, 2, and 3. The head, which is pyramidal, rounded or oval shaped, serves as a cervical expander due to the fact that this method is applied during different cycle periods and the cervix can be probably closed. Body size ranges from 1 (one) to 4 (four) centimeters in length and from 1 (one) to 4 (four) millimeters in diameter, varying according to uterus size. This body is coiled by a copper filament. Arms ranges from 3 (three) to 10 (ten) centimeters in length and from 2 (two) to 8 (eight) millimeters in diameter. There is a "spun" stainless steel bar inside the body, used as a plunger, which serves the purpose of removing the device from the inserter tube once it is located into the uterine horns. Besides two polypropylene knobs are specially designed to facilitate device handling.

Device insertion comprises the following steps:
1. The animal is immobilized by means of an animal trap and then the tail is raised.
2. The inserter tube bearing the devices therein is submerged into a receptacle containing disinfectant solution basically constituted by 10% iodine.
3.Vulvar lips are separated and the inserter tube is placed in the vaginal vestibule up to the cervix.
4. The right arm of the inserter tube is placed into the animal rectus, the cervix is strongly fixed and guided frontward by means of the thumb, index and middle fingers.
5. The end of the inserter tube composed by the device head is inserted into the cervix lengthwise in order to reach the uterine body.
6. Then one of the horns is reached and the device or devices are placed in the uterine horn bottom.
7. The inserter tube is again placed in the uterine body, the operator takes the other horn with his right hand and places the inserter tube in the other horn to insert the remaining device or devices therein.

Subsequently, the applicator is removed and the process ends.

A particularly preferred device was developed with a shape and size enough to distend the uterine cavity slightly in nuliparous heifers.

The two side arms, of 2.5 mm in diameter and 3.5 cm in length, open and expand at the distal end of the main elongated body giving a maximal anchor of 3.5 cm. In use, two devices are located into an inserter tube with an outer diameter of 0.4 cm and 44.5 cm in length. Each device with its folded arms is located immediately below the second one in the tube. The folded flexible arms of each IUD remain open as they left the tube.

## Claims

1. Intrauterine device for improving fattening in mammals with two separated uterine horns, the device comprising a main elongated body (2) which is coiled by a metallic filament and has a lower extreme being formed by two or more arms (3) which are inverted "V" shaped, **characterised in that** the main elgonated body has an upper extreme being formed by a head (1) which has an upper pyramidal, rounded or oval surface and each arm (3) is provided with a longitudinal slit.

## Patentansprüche

1. Intrauterine Vorrichtung zur Verbesserung der Mast bei Säugern mit zwei getrennten Uterushörnern, die Vorrichtung umfassend einen länglichen Hauptkörper (2), der von einem Metallfilament umwickelt ist und ein unteres Ende aufweist, das aus zwei oder mehr Armen (3) gebildet ist, die invertierte "V"-Form aufweisen, **dadurch gekennzeichnet**, dass der längliche Hauptkörper ein oberes Ende aufweist, das durch einen Kopf (1) gebildet wird, der eine obere pyramidale, gerundete oder ovale Oberfläche aufweist, und dass jeder Arm (3) mit einem longitudinalen Spalt versehen ist.

## Revendications

1. Dispositif intra-utérin destiné à améliorer l'engraissement de mammifères ayant deux trompes utérines séparées, le dispositif comportant un corps principal allongé (2) comportant un filament métallique enroulé et ayant une extrémité inférieure formée par deux ou plus de deux branches (3) qui sont en forme de "V" retourné, **caractérisé en ce que** le corps principal allongé comporte une extrémité supérieure formée par une tête (1) qui présente une surface supérieure pyramidale arrondie ou ovale et chaque branche (3) est pourvue d'une fente longitudinale.
